# EUROPÄISCHE PATENTSCHRIFT

(11) **EP 3 554 598 B1**
(45) Veröffentlichungstag und Bekanntmachung des Hinweises auf die Patenterteilung: **19.08.2020**
(21) Anmeldenummer: 17837849.3
(22) Anmeldetag: 18.12.2017
(51) Int. Cl.: A61M 13/00

(54) **MEDIZINTECHNISCHE PUMPE MIT VERBESSERTER DESUFFLATION**
MEDICAL PUMP WITH IMPROVED DESUFFLATION
POMPE TECHNIQUE MÉDICALE À DÉSUFFLATION AMÉLIORÉE

(30) Priorität: 16.12.2016 DE 102016014980
(43) Veröffentlichungstag der Anmeldung: 23.10.2019
(73) Patentinhaber: W.O.M. World of Medicine GmbH, 10587 Berlin (DE)
(72) Erfinder: KÖTH, Yves, 12683 Berlin (DE)
(74) Vertreter: Jungblut & Seuss
(86) Internationale Anmeldenummer: PCT/DE2017/000428
(87) Internationale Veröffentlichungsnummer: WO 2018/108200

(56) Entgegenhaltungen:
- WO-A1-2004/009167
- DE-A1- 4 339 876
- DE-A1-102013 016 063
- DE-A1-102015 000 845
- US-A1- 2007 249 990
- US-A1- 2016 106 934

## Beschreibung

Die vorliegende Erfindung betrifft einen Insufflator für die Laparoskopie, enthaltend eine Saugpumpe, wobei die Saugpumpe mittels eines separaten Schlauches mit dem Patienten verbunden ist und eine kontrollierte Entlüftung ermöglicht.

### Hintergrund und Stand der Technik

Die Laparoskopie ist ein medizinischer Eingriff, bei dem die Bauchhöhle und die darin liegenden Organe visuell überprüft werden können. Hierzu werden üblicherweise kleine Hautschnitte (0,3 bis 2 Zentimeter) in die Bauchdecke gemacht und durch diese ein Trokar eingebracht, welcher wiederum eine optische Vorrichtung aufnehmen kann. Mithilfe eines speziellen Endoskops (Laparoskop) kann der Bauchraum eingesehen werden. Bei der diagnostischen Laparoskopie wird der Bauchraum lediglich visuell inspiziert, im Rahmen einer therapeutischen Laparoskopie können auch operative Eingriffe vorgenommen werden.

Üblicherweise wird zu Beginn der Laparoskopie zunächst der Bauchraum mit Gas befüllt, um ein Pneumoperitoneum zu schaffen. Hierzu sind bereits verschiedene Gase verwendet worden, wie z. B. Luft, Stickstoff oder Kohlendioxid (CO₂). Die Verwendung von Kohlendioxidgas hat sich besonders gut bewährt.

Nach dem Ende der Operation muss das eingeführte Gas wieder entfernt werden (Entlüftung oder Desufflation). Dies wird heutzutage unkontrolliert durch Öffnen eines Trokaranschlusses durchgeführt. Dabei ergeben sich insbesondere folgende Probleme:
1) Das im Patienten vorhandene Gas wird ungefiltert in den Operationsraum abgelassen. Damit ist das OP-Personal direkt den giftigen Rauchgasen ausgesetzt
2) Durch die unkontrollierte Entlüftung besteht ein höheres Risiko des Verbleibs von CO₂ im Patienten. Durch die dadurch notwendige Absorption des CO₂ durch den Körper entsteht oftmals ein postoperativer Schmerz ("shoulderpain").
3) Durch die unkontrollierte Entlüftung fällt der Bauchraum unverhältnismäßig schnell zusammen.

Die im Stand der Technik beschriebenen Insufflationseinrichtungen, die Saugvorrichtungen aufweisen (z.B. DE4219859 A1 , WO 2004/ 009167 A1 oder WO 2011/041387 A1) sind aus Sicherheitsgründen nicht zur Entlüftung geeignet.

Die Aufgabe der vorliegenden Erfindung ist es, eine kontrollierte und sichere Desufflation des Patienten zu ermöglichen und die benannten Nachteile zu vermeiden.

### Lösung der Aufgabe

Die Lösung dieser Aufgabe erfolgt durch den Gegenstand der Patentansprüche, d. h. eine Insufflationseinrichtung mit Saugpumpe sowie einer Insufflations- und einer separaten Desufflationsleitung wobei sowohl in der Insufflations-, als auch in der Desufflationsleitung ein Drucksensor und ein Volumenstromsensor vorhanden ist, so dass der Druck in beiden Leitungen zeitgleich gemessen werden kann.

Die Lösung der Aufgabe erfolgt insbesondere durch einen
Insufflationseinrichtung zur Verwendung in der Medizintechnik enthaltend
einen Insufflator zur Gasversorgung mit einer Gasquelle,
eine Regelungseinheit,
eine Insufflationsleitung und eine separate Desufflationsleitung,
wobei die Desufflationsleitung mit einer Saugpumpe verbunden ist,
wobei die Insufflationsleitung und die Desufflationsleitung je einen Drucksensor und je einen Volumenstromsensor aufweisen,
wobei der Insufflator eine Vorrichtung zur kontrollierten Absaugung des im Patienten befindlichen Gases aufweist
wobei die Regelungseinheit die Leistung der Saugpumpe in Abhängigkeit von der Druckmessung des Drucksensoren steuert,
wobei die Regelungseinheit eine Aktivierungssperre enthält, die eine Aktivierung der Saugpumpe verhindert, wenn der mittels des Drucksensors in der Insufflationsleitung gemessene Druck kleiner als ein eingestellter Schwellwert ist,
wobei die Aktivierungssperre ferner eine Aktivierung der Saugpumpe verhindert, wenn der mittels des Drucksensors in der Insufflationsleitung gemessene Druck und der mittels des Drucksensors in der Absaugleitung gemessene Drucks nicht identisch sind

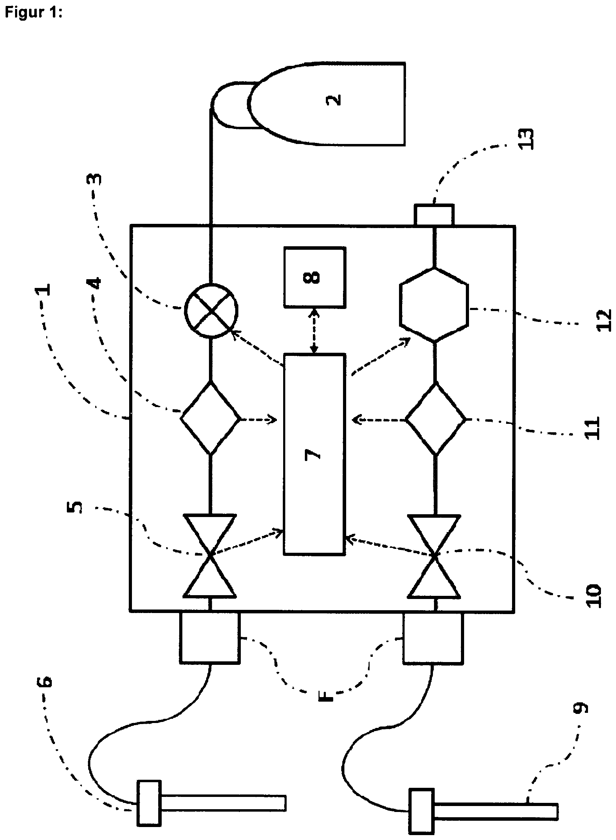

Um eine Desufflation zu ermöglichen, wird der Insufflator unter Zuhilfenahme zweier Schläuche mit dem Patienten verbunden. Der erste Schlauch wird zur Insufflation verwendet. Gas wird im Rahmen der Operation dem Patienten zugeführt, um den Druck im Abdomen aufbauen zu können. Weiterhin wird die abdominale Druckmessung über diese Leitung durchgeführt. Der zweite Schlauch kann bereits während der Durchführung der Operation mit dem Patienten verbunden werden, um beispielsweise eine Rauchgasabsaugung zu realisieren (Figur 1).

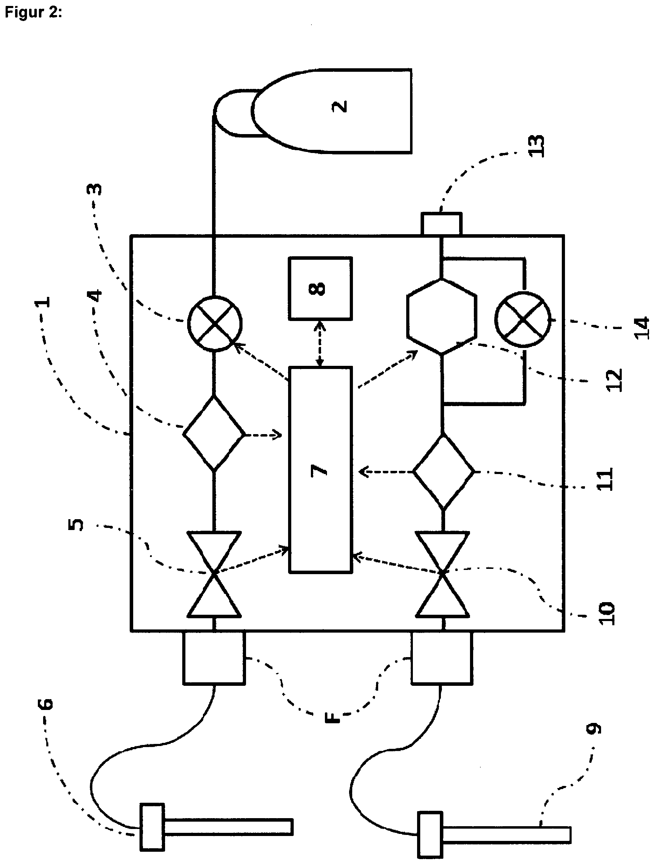

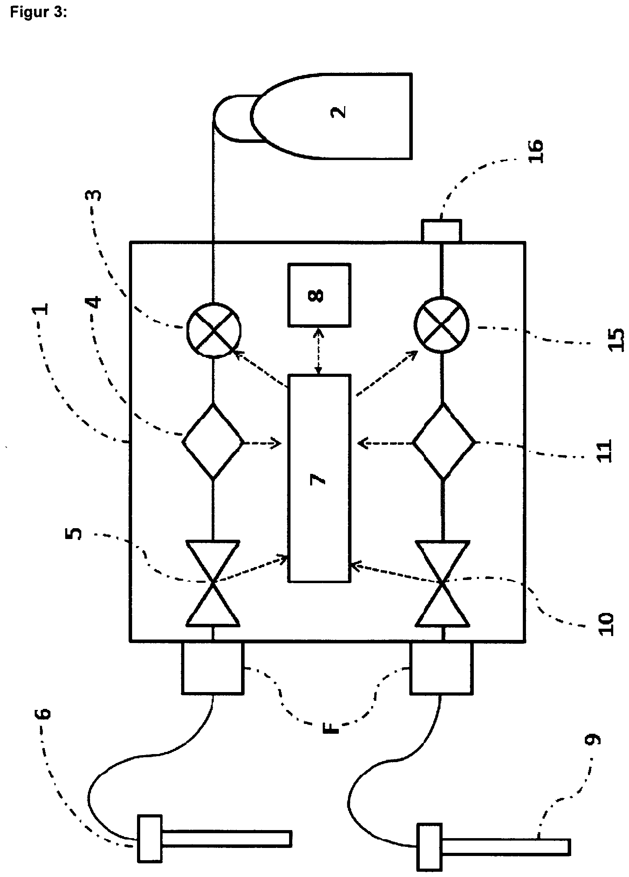

Als Absaugpumpen kommen elektronisch geregelte Pumpen in Frage, wie sie beispielsweise in der Vorrichtung gemäß DE 102013016063 oder ähnlichen Druckschriften beschrieben sind.
Alternativ kann beispielsweise die Saugpumpe über ein ByPass-Ventil gesteuert werden (Figur 2). Hierzu kann beispielsweise die Pumpe auf eine bestimmte Leistung eingestellt werden, welche weitestgehend konstant ist, und die Regelung der Leistung erfolgt dann über das ByPass-Ventil.
Weiterhin alternativ kann auch ein Steuerungsventil direkt in die Absaugleitung positioniert werden (Figur 3). Auf diese Weise könnten sogar externe Pumpen verwendet werden, beispielsweise die im OP vorhandene Wandabsaugung. Die Regelungseinheit des Insufflators regelt dann die Absaugleistung über das dargestellte Steuerungsventil.

Nach dem Stopp der Insufflation wird durch den Insufflator dem Benutzer die Möglichkeit gegeben, die Desufflation zu starten. Beim Start des Desufflationsvorgangs prüft der Insufflator zunächst den gemessenen Abdominaldruck über die Insufflationsleitung. Ist dieser geringer als ein einstellbarer Schwellwert (<5 mmHg, bevorzugt < 3 mmHg), bewirkt eine Aktivierungssperre, dass die Desufflation nicht gestartet wird. Der genannte Schwellwert kann geräteseitig voreingestellt sein. In besonderen Ausführungsformen der Erfindung kann ein Wählschalter oder eine andere Wähleinrichtung vorhanden sein, mit der der Schwellwert präoperativ vom Bedienpersonal eingestellt werden kann. Liegt der gemessene Abdominaldruck über dem eingestellten Schwellwert, dann wird der über die Insufflationsleitung gemessene Abdominaldruck mit dem gemessenen Druck auf der Absaugleitung verglichen. Ist dieser identisch kann davon ausgegangen werden, dass beide Schläuche mit dem Patienten verbunden sind. Die beiden gemessenen Druckwerte werden als identisch angesehen, wenn ihre Differenz kleiner ist als 2 mmHg.

Sind diese Voraussetzungen erfüllt, kann die Saugpumpe aktiviert werden.Die Absaugrate darf aus Sicherheitsgründen nicht zu hoch sein, sie sollte aber auch nicht zu gering sein, um die Behandlung nicht unnötig in die Länge zu ziehen. Als sinnvoll hat sich eine Absaugrate von 1-5 I/ min. bevorzugt ca. 3 I/min. erwiesen.

Der abdominale Druck wird weiterhin über die Insufflationsleitung überwacht. Sobald der abdominale Druck unterhalb einer bestimmten Grenze liegt (z.B. 5, 4 oder 3 mmHg) wird die Desufflation gestoppt. Die Druckgrenze (von z.B. 5, 4 oder 3 mmHg) kann geräteseitig voreingestellt sein. Üblicherweise ist ein Wählschalter oder eine andere Wähleinrichtung vorhanden, mit der die Druckgrenze vor der Operation vom Bedienpersonal eingestellt werden kann.

Wenn während der Desufflation die Insufflationsleitung durch einen Verschluss (z.B. durch Schließen des Stopcocks am Trokar) oder durch fehlende Verbindung (z. B. frühes Entfernen des Trokars) den abdominalen Druck nicht mehr korrekt messen kann, besteht die Gefahr einer zu späten Abschaltung der Desufflation und damit eines negativen Druckes im Patienten.

Im Patent Northgate US Pat 6,299,592 wird ein Verfahren beschrieben, wie ein Verschluss in der Insufflationsleitung erkannt werden kann. Bei diesem erfolgt jedoch eine ständige Insufflation in der Insufflationsleitung. Da ein zusätzliches Zuführen von Gas während des Absaugens kontraproduktiv ist, wird im Folgenden ein anderes Verfahren dargestellt um die Sicherheit zu gewährleisten.

Im Rahmen der erfindungsgemäßen Vorrichtung wird die Desufflation intervallartig unterbrochen. Nach dem Abschalten der Saugpumpe wird jeweils eine gewisse Zeit gewartet, bis der Druckausgleich stattgefunden hat. Dieser ist u.a. abhängig vom Strömungswiderstand und je nach Anwendungsfall variabel. Um zum Einen zügig den abdominalen Druck zu reduzieren und zum Anderen die Wartezeit zum Druckausgleich zu gewährleisten, sollte bevorzugt dynamisch gewartet werden. Das Ende der Wartezeit ist erreicht, wenn ein stabiler Druck auf der Saugseite vorliegt. Es wird anschließend geprüft, ob der gemessene Abdominaldruck auf der Insufflationsleitung dem gemessenen Druck in der Desufflationleitung entspricht. Ist dies nicht der Fall, wird die Desufflation gestoppt. Andernfalls wird die Desufflation fortgesetzt.

Zu Beginn der Desufflation beträgt das Zeitintervall, in welchem gesaugt wird beispielsweise zwischen 10 und 1 Sekunden, besonders bevorzugt 5-3 Sekunden. Das Messintervall beträgt typischerweise zwischen 5 und 0,5 Sekunden.

Sollte eine größere Leckage im Bereich des Abdomens (z.B. durch einen weiteren Trokar) oder ein kleines Volumen im Abdomen vorhanden sein, besteht jedoch weiterhin die Gefahr, einen Unterdruck im Patienten zu erzeugen.Der oben beschriebene Algorithmus erkennt nur in festen Intervallen (z.B. alle 3 Sekunden) den Gleichlauf der beiden Drucksensoren. Dieses Intervall ist für diese Fälle zu lang, um sicher einen Unterdruck zu erkennen. Eine Verkürzung der Intervalle führt zu einer unzureichenden Absaugrate.

Aus diesem Grund muss während der aktiven Absaugung ein Plausibilitätstest durchgeführt werden. Sinkt der Abdominaldruck im Verhältnis zum abgesaugten Volumen während des Absaugvorgangs zu langsam (z.B. größer 0,3 Liter/mmHg als Erwartungswert für einen durchschnittlichen Patienten) wird die Desufflation unterbrochen. Dieses Volumen wird mit dem vorhandenen Sensor zur Volumenstrommessung bestimmt.

In einer bevorzugten Ausführungsform der Erfindung wird während der Insufflation des Patienten der abdominale Druckanstieg ermittelt, in ein Verhältnis zum insufflierten Volumen gesetzt und gespeichert. Auf diese Weise kann beispielsweise ermittelt werden, dass für einen Druckanstieg von 1 mmHg ein Gasfluss von 0,35 Liter erforderlich ist. Dieser Wert wird in der Regelungseinheit gespeichert. Für die Desufflation desselben Patienten ist zu erwarten, dass pro 0,35 Liter abgepumpten Gases ein Druckabfall von 1 mmHg eintritt (Erwartungswert: 0,35l/mmHg). Während der Desufflation wird der Druckabfall pro Gasvolumen überwacht. Sollte der Erwartungswert signifikant über- oder unterschritten werden (z.B. um mehr als 20%) wird die Desufflation automatisch gestoppt.

Alternativ kann eine kontinuierliche Überprüfung des abdominalen Drucks mit Hilfe des Drucksensors auf der Saugseite erfolgen. Diese Überprüfung basiert auf einem mathematischen Beobachtermodell (z.B. Luenberger-Beobachter), der auf Basis des Modells der Regelstrecke entworfen wurde. Derartige Modelle sind im Stand der Technik beschrieben (WO 2016/119773 A1) und benötigen daher keine weiteren Erläuterungen an dieser Stelle. Auf diese Weise kann während der Absaugung bereits ein Gleichlauf der beiden Drucksensoren überwacht und bei Abweichung die Desufflation unterbrochen werden.

Neben einem Verschluss in der Insufflationsleitung muss auch ein Verschluss in der Saugleitung erkannt werden, um keine Gewebeschäden zu verursachen (z. B. Ansaugen von Organen). Auf der Saugseite wird bei der Reduzierung des abgesaugten Volumenstroms oder bei zu hohem Unterdruck ein Verschluss auf Saugseite erkannt und die Desufflation ebenfalls unterbrochen. Es wird in diesen Fällen anschließend geprüft, obder gemessene Abdominaldruck auf der Insufflationsleitung dem gemessenen Druck in der Desufflationleitung entspricht. Ist dies nicht der Fall, wird die Desufflation gestoppt. Andernfalls wird die Desufflation fortgesetzt.

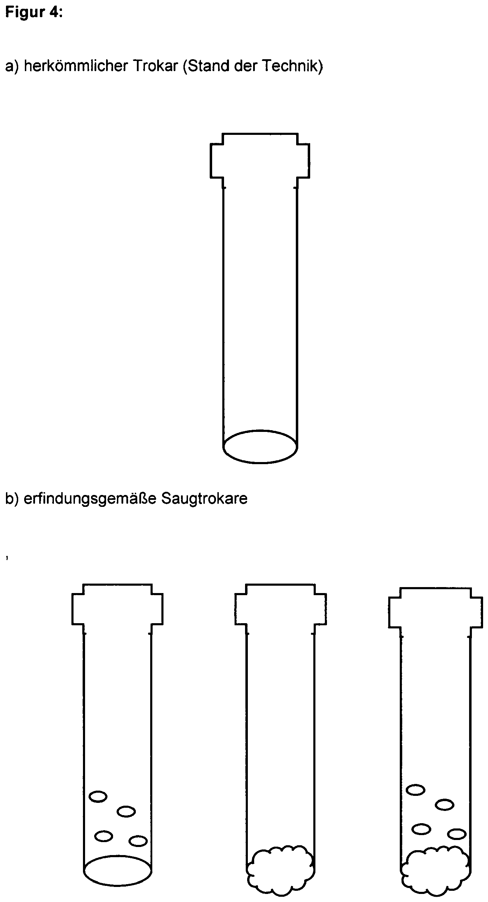

Ergänzend kann die Absaugung mittels spezieller Saugtrokare erfolgen. Klassische Trokare sind im Wesentlichen stiftförmige (zylinderförmige) Rohre mit einer kreisrunden Öffnung, die im Anwendungsfall im Bauchraum des Patienten positioniert ist, und diversen Anschlussmöglichkeiten außerhalb des Patienten. Bei der Absaugung von Gas im Rahmen der Desufflation besteht die Gefahr, dass sich die Ansaugöffnung an empfindlichen Gewebeteilen festsaugt. Diese Gefahr wird verringert wenn die Trokare so ausgestaltet sind, dass die Zylinderwand im Endteil Öffnungen aufweisen. In einer bevorzugten Ausführungsform enthält der Trokar im Bereich der Öffnung Belüftungsausnehmungen (Figur 4).

Figur 1 zeigt eine Ausführungsform eines erfindungsgemäßen Insufflators. Der Insufflator (1) ist an eine Gasquelle (2), z.B. in Form einer CO₂-Gasflasche angeschlossen. Über ein Proportionalventil (3), einen Drucksensor (4), einen Volumenstromsensor (5) und einen Filter (F) dem Insufflationstrokar (6) zugeführt. Der separate Desufflationstrokar (9) ist über einen Schlauch mit dem Insufflator verbunden, wobei der Gasflusszunächst wieder über einen Filter (F) einen Volumenstromsensor (10) einem Drucksensor (11) zu einer Saugpumpe (12) führt. Der Ausgang der Saugpumpe führt zu einem Geräteauslass (13). Der Geräteauslass (13) kann selbstverständlich mit einem zusätzlichen Filter ausgestattet sein. Die Messdaten der Drucksensoren (4, 11) sowie der Volumenstromsensoren (5,10) werden an die Recheneinheit (7) mit angeschlossenem Speicher (8) übermitteilt. Die Recheneinheit (7) steuert das Proportionalventil (3) sowie die Saugpumpe (12). Wie der Fachmann erkennen wird, können die Positionen der Drucksensoren und der Volumenstromsensoren auch anders lokalisiert sein: So ist es selbstverständlich möglich, dass der Insufflationsstrom zunächst durch den Volumenstromsensor (5) und dann durch den Drucksensor (4) geführt wird. Ähnlich ist es möglich, dass der Volumenstromsensor (10) der Desufflationsleitung in Strömungsrichtung erst nach der Saugpumpe (12) liegt.

Figur 2 zeigt eine erfindungsgemäße Vorrichtung, bei der die Saugpumpe (12) kontinuierlich läuft und die Absaugleistung durch ein By-Pass-Ventil (14) gesteuert wird. Das By-Pass-Ventil (14) wird ebenfalls über die Recheneinheit (7) gesteuert (in der Figur 2 nicht dargestellt).

Figur 3 zeigt eine weitere Variante der erfindungsgemäßen Vorrichtung. Hierbei wird statt einer geräteinternen Saugpumpe ein Anschluss (16) für eine externe Pumpe vorgesehen. Viele Krankenhäuser sind mit entsprechenden Pumpen ausgestattet, die für den vorgesehenen Einsatz eines erfindungsgemäßen Insufflators verwendet werden können. In diesem Fall wird lediglich ein Steuerungsventil (15) benötigt, um die Saugrate der externen Pumpe (nicht dargestellt) zu regulieren.

Figur 4 zeigt Ausführungsformen von Saugtrokaren. Figur 4a zeigt einen herkömmlichen (zylinderförmigen) Trokar mit entsprechenden Anschlüssen. Figur 4b zeigt verschiedene erfindungsgemäße Saugtrokare, die entweder Öffnungen in der Zylinderwand aufweisen (links) oder im Bereich des Zylinderendes unregelmäßig ausgestaltet sind (Mitte) oder sowohl Öffnungen in der Zylinderwand, wie auch eine unregelmäßig geformtes Endstück aufweisen (rechts). Entscheidend hierbei ist, dass das Ende des Zylinders nicht auf Gewebe aufsitzen und sich daran festsaugen kann. Durch die vorgesehenen Öffnungen wird ein derartiges Festsaugen wirkungsvoll verhindert.

Die Einzelkomponenten der erfindungsgemäßen Vorrichtung sind größtenteils bereits aus früheren Druckschriften bekannt wie z.B. US 6299592, US5411474, WO1996001132A1, WO 2011041387A1, US 5800381, DE 4219859B4, DE 102015000845A1, jedoch nicht im Zusammenhang mit einer erfindungsgemäßen Vorrichtung, wie sie durch die Ansprüche definiert sind. Als Regelungseinheit dient ein entsprechend programmierter Microcomputer mit dazugehörigen Speicher sowie Eingabe-und Ausgabevorrichtungen. Volumenstromsensoren sind bereits aus anderen medizintechnischen Vorrichtungen bekannt (z.B. im Rahmen von Beatmungsvorrichtungen), so dass sie an dieser Stelle nicht weiter erörtert werden müssen.

Der Fachmann auf dem Gebiet kann alternative und/oder ergänzende Ausführungsformen der Erfindung realisieren, ohne erfinderisch tätig werden zu müssen.

## Patentansprüche

1. Insufflationseinrichtung zur Verwendung in der Medizintechnik enthaltend
einen Insufflator (1) zur Gasversorgung mit einer Gasquelle (2),
eine Regelungseinheit (7),
eine Insufflationsleitung mit einem Trokar (6) und eine separate Desufflationsleitung mit einem Trokar (9),
wobei die Desufflationsleitung mit einer Saugpumpe (12) verbunden ist,
wobei die Insufflationsleitung und die Desufflationsleitung je einen Drucksensor (4, 11) und je einen Volumenstromsensor (5, 10) aufweisen,
wobei der Insufflator eine Vorrichtung zur kontrollierten Absaugung des im Patienten befindlichen Gases aufweist
wobei die Regelungseinheit (7) die Leistung der Saugpumpe (12) in Abhängigkeit von der Druckmessung des Drucksensoren (4, 11) steuert,
**dadurch gekennzeichnet, dass** die Regelungseinheit (7) eine Aktivierungssperre enthält, die eine Aktivierung der Saugpumpe (12) verhindert, wenn der mittels des Drucksensors (4) in der Insufflationsleitung gemessene Druck kleiner als ein eingestellter Schwellwert ist,
wobei die Aktivierungssperre ferner eine Aktivierung der Saugpumpe (12) verhindert, wenn der mittels des Drucksensors (4) in der Insufflationsleitung gemessene Druck und der mittels des Drucksensors (11) in der Absaugleitung gemessene Drucks nicht identisch sind.

2. Insufflationseinrichtung zur Verwendung in der Medizintechnik gemäß Anspruch 1, wobei der Schwellwert kleiner als 5 mmHg ist.

3. Insufflationseinrichtung zur Verwendung in der Medizintechnik gemäß Anspruch 1, wobei der Schwellwert kleiner als 3 mmHg ist.

4. Insufflationseinrichtung zur Verwendung in der Medizintechnik gemäß mindestens einem der Ansprüche 1 bis 3, wobei die Regelungseinheit (7) die Saugpumpe (12) zyklisch deaktiviert, und während der Saugpausen, die Druckwerte der beiden Drucksensoren (4,11) vergleicht, wobei nur bei identischen Werten die Saugpumpe wieder aktiviert wird.

5. Insufflationseinrichtung zur Verwendung in der Medizintechnik gemäß mindestens einem der Ansprüche 1 bis 4, wobei die Regelungseinheit (7) das abgesaugte Volumen ins Verhältnis zum sinkenden abdominalen Druck setzt und wobei die Regelungseinheit (7) die Saugpumpe (12) deaktiviert, wenn dieses Verhältnis nicht einem Erwartungswert entspricht.

6. Insufflationseinrichtung zur Verwendung in der Medizintechnik gemäß Anspruch 5, wobei die Regelungseinheit (7) den Erwartungswert des Verhältnisses von abgesaugtem Volumen zu sinkenden abdominalen Druck während der Insufflationsphase durch Messung des abdominalen Druckanstiegs zum Verhältnis des insufflierten Volumens während der Insufflationsphase ermittelt und speichert.

7. Insufflationseinrichtung zur Verwendung in der Medizintechnik gemäß mindestens einem der Ansprüche 1 bis 5, wobei die Regelungseinheit die Saugpumpe kontinuierlich aktiviert, und den Druck des Bauchraumes über den Drucksensor (11) der Desufflationsleitung mittels eines mathematischen Beobachtermodell schätzt und mit dem Druck des Drucksensors in der Insufflationsleitung (4) vergleicht, wobei nur bei identischen Werten die Saugpumpe (12) aktiviert bleibt.

8. Insufflationseinrichtung zur Verwendung in der Medizintechnik gemäß Anspruch 7, wobei das mathematische Beobachtermodell nach Art eines Luenberger-Beobachters ausgestaltet ist.

9. Insufflationseinrichtung zur Verwendung in der Medizintechnik gemäß mindestens einem der Ansprüche 1 bis 4, wobei die Desufflationsleitung mit einem zylinderförmigen Trokar (9) verbunden, wobei der zylinderförmige Trokar so ausgestaltet ist, dass er Ausnehmungen in der Zylinderwand aufweist.

## Claims

1. The insufflation device for use in medical engineering containing an insufflator (1) for gas supply with a gas source (2),
a control unit (7),
an insufflation conduit with a trocar (6) and a separate desufflation conduit with a trocar (9),
the desufflation conduit being connected to a suction pump (12),
the insufflation conduit and the desufflation conduit including one pressure sensor (4, 11) and one volume flow sensor (5, 10) each,
the insufflator including a device for controlled extraction of the gas present in the patient,
the control unit (7) controlling the power of the suction pump (12) depending on the pressure measurement of pressure sensors (4, 11),
**characterized by** that the control unit (7) includes an activation blocking element preventing an activation of the suction pump (12), when the pressure in the insufflation conduit measured by means of the pressure sensor (4) is smaller than a set threshold value,
wherein the activation block further prevents an activation of the suction pump (12), when the pressure in the insufflation conduit measured by means of the pressure sensor (4) and the pressure in the extraction conduit measured by means of the pressure sensor (11) are not identical.

2. The insufflation device for use in medical engineering according to claim 1, wherein the threshold value is smaller than 5 mm Hg.

3. The insufflation device for use in medical engineering according to claim 1, wherein the threshold value is smaller than 3 mm Hg.

4. The insufflation device for use in medical engineering according to at least one of claims 1 to 3, wherein the control unit (7) cyclically deactivates the suction pump (12), and during the suction pauses, compares the pressure values of the two pressure sensors (4, 11), only with identical values the suction pump being activated again.

5. The insufflation device for use in medical engineering according to at least one of claims 1 to 4, wherein the control unit (7) proportions the extracted volume to the decreasing abdominal pressure, and wherein the control unit (7) deactivates the suction pump (12), when this proportion does not correspond to an expected value.

6. The insufflation device for use in medical engineering according to claim 5, wherein the control unit (7) determines the expected value of the proportion of the extracted volume to decreasing abdominal pressure during the insufflation phase by measurement of the abdominal pressure rise in proportion to the insufflated volume during the insufflation phase and stores it.

7. The insufflation device for use in medical engineering according to at least one of claims 1 to 5, wherein the control unit continuously activates the suction pump, and estimates the pressure of the abdomen via the pressure sensor (11) of the desufflation conduit by means of a mathematical observer model and compares it to the pressure of the pressure sensor in the insufflation conduit (4), only with identical values the suction pump (12) remaining activated.

8. The insufflation device for use in medical engineering according to claim 7, wherein the mathematical observer model is arranged in the manner of a Luenberger observer.

9. The insufflation device for use in medical engineering according to at least one of claims 1 to 4, wherein the desufflation conduit is connected to a cylindrical trocar (9), the cylindrical trocar being arranged such that it includes recesses in the cylinder wall.

## Revendications

1. Dispositif d'insufflation utile pour application médicale comportant un insufflateur (1) for alimentation en gaz avec une source de gaz (2),
une unité de régulation (7),
un conduit d'insufflation avec un trocar (6) et un conduit de désufflation séparé avec un trocar (9),
le conduit de désufflation étant connecté à un pompe aspirante (12),
le conduit d'insufflation et le conduit de désufflation comportant un capteur de pression (4,11) et un capteur de débit volumique (5, 10) chacun,
l'insufflateur comportant un dispositif pour l'extraction contrôlée du gaz présent dans le patient,
l'unité de régulation (7) commandant la puissance de la pompe aspirante (12) selon la pression mesurée par les capteurs de pression (4, 11),
**caractérisé en ce que** l'unité de régulation (7) comporte un blocage d'activation empêchant une activation de la pompe aspirante (12), si la pression dans le conduit d'insufflation mesurée au moyen du capteur de pression (4) est inférieure à une valeur de seuil ajustée,
dans lequel le blocage d'activation en outre empêche une activation de la pompe aspirante (12), si la pression dans le conduit d'insufflation mesurée au moyen du capteur de pression (4) et la pression dans le conduit d'extraction mesurée au moyen du capteur de pression (11) ne sont pas identiques.

2. Dispositif d'insufflation utile pour application médicale selon la revendication 1, dans lequel la valeur de seuil est inférieure à 5 mm Hg.

3. Dispositif d'insufflation utile pour application médicale selon la revendication 1, dans lequel la valeur de seuil est inférieure à 3 mm Hg.

4. Dispositif d'insufflation utile pour application médicale selon au moins une des revendications 1 à 3, dans lequel l'unité de régulation (7) cyclique-ment désactive la pompe aspirante (12), et pendant les pauses de l'aspiration, compare les valeurs de pression des deux capteurs de pression (4, 11), seulement avec des valeurs identiques la pompe aspirante étant activée de nouveau.

5. Dispositif d'insufflation utile pour application médicale selon au moins une des revendications 1 à 4, dans lequel l'unité de régulation (7) met le volume extrait au proportion à la pression abdominale décroissante, et dans lequel l'unité de régulation (7) désactive la pompe aspirante (12), si cette proportion ne correspond pas à une valeur espérance.

6. Dispositif d'insufflation utile pour application médicale selon la revendication 5, dans lequel l'unité de régulation (7) détermine la valeur espérance de la proportion du volume extrait à la pression abdominale décroissante pendant la phase d'insufflation par mesurage de l'augmentation de la pression abdominale en proportion au volume insufflé pendant la phase d'insufflation et la mémorise.

7. Dispositif d'insufflation utile pour application médicale selon au moins une des revendications 1 à 5, dans lequel l'unité de régulation active en continue la pompe aspirante, et estime la pression de l'abdomen par l'intermédiaire du capteur de pression (11) du conduit de désufflation au moyen d'un modèle d'observateur mathématique et la compare à la pression du capteur de pression dans le conduit d'insufflation (4), seulement avec des valeurs identiques la pompe aspirante (12) restant activée.

8. Dispositif d'insufflation utile pour application médicale selon la revendication 7, dans lequel le modèle d'observateur mathématique est arrangé de façon d'un observateur Luenberger.

9. Dispositif d'insufflation utile pour application médicale selon au moins une des revendications 1 à 4, dans lequel le conduit de désufflation est connecté à un trocar cylindrique (9), le trocar cylindrique étant arrangé de façon qu'il comporte des évidements dans la paroi cylindrique.
